# EUROPEAN PATENT SPECIFICATION

(11) **EP 0 544 654 B1**
(45) Date of publication and mention of the grant of the patent: **07.12.1994**
(21) Application number: 93200504.4
(22) Date of filing: 23.01.1989
(51) Int. Cl.: A61M 39/00

(54) **Blunt cannula device**
Stumpfkanülenvorrichtung
Dispositif de canule émoussée

(30) Priority: 25.01.1988 US 147414; 08.07.1988 US 217004
(43) Date of publication of application: 02.06.1993
(62) Divisional of application: 89902147.1
(73) Proprietor: BAXTER INTERNATIONAL INC., Deerfield, Illinois 60015 (US)
(72) Inventor: Jepson, Steven C., Palatine, Illinois 60067 (US); Dudar, Thomas E., Palatine Illinois 60067 (US); Zdeb, Brian D., Round Lake, Illinois 60073 (US); Desecki, Vince C., Ingleside, Illinois 60041 (US)
(74) Representative: MacGregor, Gordon

(56) References cited:
- EP-A- 0 050 459
- EP-A- 0 114 677
- FR-A- 2 439 022

## Description

This invention relates to a blunt cannula device for use with an injection site having a pre-slit septum.

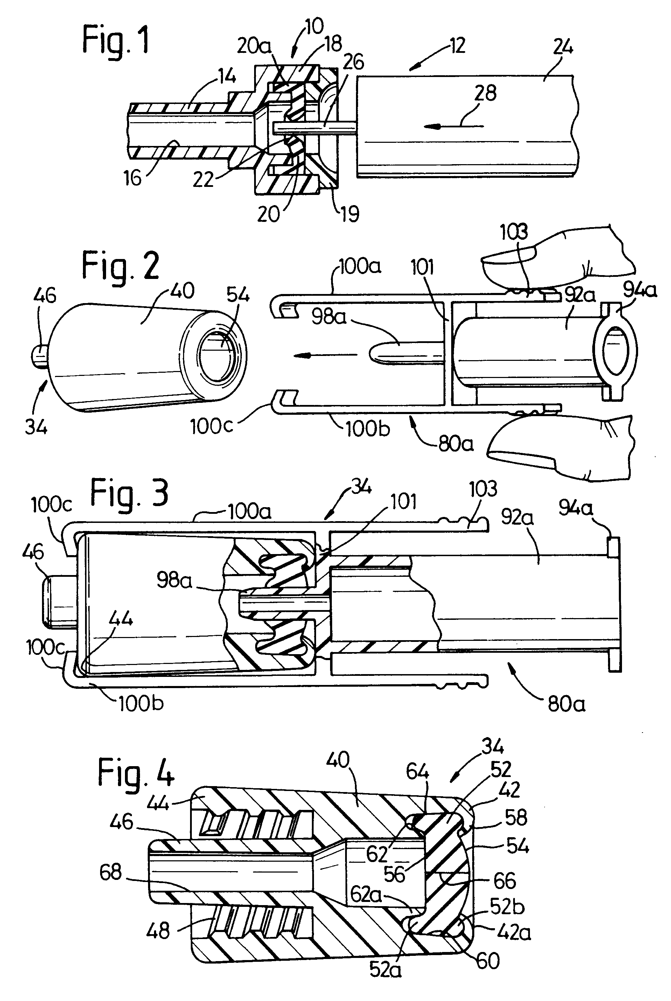

A blunt cannula device is known, as disclosed with respect to Fig. 1 of the drawings, comprising a body portion and a hollow, elongate cylindrical, blunt-ended piercing member extending from the body portion.

Use of a blunt cannula is also disclosed in US-A-4197848.

The present invention provides a blunt cannula device, whose piercing member cannot accidentally disengage from the septum in use.

EP-A-0114677 discloses a connector comprising a tubular body portion, in which a plug carries a pointed needle. The body portion has external projections which carry deflectable locking arms for engagement with an injection site when the needle is inserted into a septum of the site. The arms prevent accidental disengagement of the needle from the septum.

The precharacterising part of Claim 1 is based on the disclosure of this document.

The distinguishing features of the invention are as set out in the characterising part of claim 1.

The cannula device of the invention may have a simple, one-piece design of plastics material.

Reference is now made to the accompanying drawings, wherein:-
Fig.1 is a side elevational view, partly in section, of a prior art injection site on an associated blunt cannula device;
Fig.2 is a perspective view of a blunt cannula device according to the invention, together with a pre-slit injection site;
Fig.3 is an enlarged side elevational view, partly broken away, illustrating the interrelationship between the injection site and blunt cannula device shown in Fig.2; and
Fig 4 is an enlarged side elevational cross-sectional view of the injection site of Figs. 2 and 3.

### Detailed Description of the Preferred Embodiments

A prior art pre-slit injection site 10 and associated blunt cannula 12 are illustrated in Fig. 1. The prior art injection site 10 has a cylindrical housing 14 with a fluid flow path 16 therethrough. A first end 18 of the housing 14 is closed with a relatively thin disc-shaped resealable member 20. The member 20 has a resealable opening 22 therein.

The member 20 is a molded septum with an integrally formed skirt 20a. The skirt 20a is oriented generally perpendicular to the portion of the septum with the opening 22.

The cannula 12 includes a body portion 24 which carries at a first end a hollow, cylindrical, blunt piercing member 26. As the cannula 12 is moved in a direction 28 toward the first end 18 of the injection site 10, the member 26 slidably engages the opening 22. The sealing member 20 is then deformed adjacent the opening 22 and the number 26 extends into the flow path 16. A fluid flow path through the cannula 12 will then be in fluid flow communication with the flow path 16 via the hollow piercing member 26.

Figs. 2 and 3 illustrate a pre-slit injection site 34 used in combination with a blunt cannula device 80a, according to the present invention.

The injection site 34 is shown in more detail in Fig.4 and comprises a cylindrical housing 40 having a first end 42 and a second end 44. The housing carries, adjacent the second end, a hollow cylindrical fluid flow member 46 for engaging a catheter connector. Internal, male luer-type threads 48 are carried by the housing 40 adjacent the second end to couple the injection site to a catheter connector. The injection site has a resealable septum 52 is carried by the first end 42 of the housing 40. The septum 52 includes first and second spaced apart surfaces 54 and 56 respectively. The surface 54 has been forced into a dome-like shape by annular, U-shaped, swaged end members 58 carried by the first end 42. The dome-like shape of the surface 54 can extend beyond a surface 42a of the first end 42. This facilitates cleaning the surface 54.

The septum 52 has a generally cylindrical shape. The septum 52 can be formed of a latex or synthetic rubber material. Alternately, the septum can be formed of a thermoplastic elastomer. The material used for the septum 52 should be non-toxic and sterilizable such as by means of radiation, steam or Ethylene Oxide.

Because the septum 52 is generally cylindrical in shape, it can be die-cut from a sheet, cut from an extruded rod or molded. The septum 52 can have an exemplary diameter on the order of .30 inches (0.762 centimeters). The height of the septum 52 can be, for example, on the order of .125 inches (.3175 centimeters).

The first end 42 is also formed with a tapered interior surface 60 which terminates in an annular channel 62. The tapered interior surface 60 has a taper in a range of 5 degrees to 20 degrees. Preferably, the taper will be on the order of 12 degrees. With the indicated size of the above noted exemplary septum 52 and a 12 degree taper, diametric resealing compression of the septum 52 adjacent the channel 62 is on the order of 10%.

The channel 62 is bounded in part by a septum supporting ridge 62a. The channel 62 can typically have a depth in a range of .050-.070 inches (.127-.1778 centimeters).

A peripheral surface 64 of the septum 52 slidably engages the tapered interior surface 60 as the septum 52 slides into the first end 42. The annular channel 62 which underlies the interior peripheral surface 56 of the septum 52 is provided to permit the septum 52 to deform when a blunt cannula is inserted through an opening 66 therein.

The housing 40 is also formed with a fluid flow path 68 such that fluids injected via a blunt cannula inserted through the resealable opening 66 can flow into the catheter 36 for delivery to hand H of the patient.

The swaged end members 58 apply axial forces to the septum 52 thereby creating the domed exterior peripheral surface 54. The axial forces applied by the end members 58 slightly deform the regions 52a and 52b. In contradistinction, the tapered internal surface 60 applies radially directed forces to the septum 52, thereby forcing the opening 66 into a resealed condition.

In an alternate embodiment, the surface 52 could be formed as a flat, as opposed to a domed, surface.

The cannula device 80a includes a tubular, hollow body portion 92a with a luer flange 94a, a piercing member 98a, and a pair of manually operable locking members comprising elongate arms 100a, 100b. The piercing member has a tapered end portion adjacent its blunt end. A cross-member 101 is located between the body portion 92a and the piercing member 98a and extends radially of the body portion and piercing member.

The elongate arms 100a, 100b are fixed between their ends to opposite ends of the cross-member 101. The arms define spring-like deflecting members.

One end portion of each arm defines manually grippable means 103 and the opposite end of each arm has a curved end region 100c, which is hook-like, as shown in Fig.2. These end regions 100c of the arms 100a, 100b, slidably engage the housing 40, when the piercing member 98a is forced through the pre-formed opening 66, and engage with the second end 44 of the housing, as shown in Fig.3. The piercing member 98a cannot, therefore, accidentally disengage from the pre-slit septum 34 during a fluid infusion process.

The injection site and the piercing member of the cannula device may have various constructions as fully described in EP-A-0354947.

## Claims

1. A cannula device for use with an injection site (34), having a septum (52) the device comprising a tubular body portion (92a), a hollow, elongate, cylindrical piercing member (98a) and a pair of locking members (100a, 100b), each locking member (100a, 100b) being a manually operable, spring-like deflectable arm having manually grippable means (103) at one end and means (100c) at the opposite end for engagement with the injection site when the piercing member is inserted into the septum, to prevent accidental disengagement of the piercing member from the septum, characterised by a cross-member (101) having the body portion (92a) extending from one side thereof and the piercing member (98a) extending from the opposite side thereof, such that the cross-member (101) extends radially between the body portion and the piercing member, each locking member (100a, 100b) being fixed between its ends to a respective end of the cross-member (101), and the piercing member (98a) having a blunt end for insertion into a pre-slit septum.

2. A blunt cannula device according to Claim 1, wherein the means (100c) for engaging the injection site comprises a curved, hook-like end region of each locking member (100a).

3. A blunt cannula device according to Claim 1, or 2, wherein the piercing member has a tapered end portion adjacent its blunt end.

4. A blunt cannula device according to any preceding claim, made in one-piece from plastics material.

5. A blunt cannula device according to any preceding claim, in combination with an injection site (34) having a first end provided with the septum (52) and a second end (44) with which the locking members are engageable.

## Patentansprüche

1. Kanüleneinrichtung zur Verwendung mit einer Injektionsstelle (34), die ein Septum (52) hat, wobei die Einrichtung folgendes aufweist: einen rohrförmigen Körperbereich (92a), ein hohles, langgestrecktes, zylindrisches Durchdringungselement (98a) und ein Paar von Arretierelementen (100a, 100b), wobei jedes Arretierelement (100a, 100b) ein manuell betätigbarer, federartiger auslenkbarer Arm ist, der eine von Hand greifbare Einrichtung (103) an dem einen Ende und eine Einrichtung (100c) an dem entgegengesetzten Ende hat, um mit der Injektionsstelle in Eingriff zu gelangen, wenn das Durchdringungselement in das Septum eingeführt wird, um zu verhindern, daß in unbeabsichtigter Weise das Durchdringungselement mit dem Septum außer Eingriff kommt,
gekennzeichnet durch
ein Querelement (101), von dem sich der Körperbereich (92a) von der einen Seite aus und das Durchdringungselement (98a) von der entgegengesetzten Seite aus erstreckt, so daß das Querelement (101) in Radialrichtung zwischen dem Körperbereich und dem Durchdringungselement verläuft, wobei jedes Arretierelement (100a, 100b) zwischen seinen Enden an einem jeweiligen Ende des Querelements (101) befestigt ist und das Durchdringungselement (98a) ein stumpfes Ende zum Einführen in ein vorgeschlitztes Septum hat.

2. Stumpfe Kanüleneinrichtung nach Anspruch 1,
wobei die Einrichtung (100c) zum Eingriff mit der Injektionsstelle einen gebogenen, hakenartigen Endbereich für jedes Arretierelement (100a) aufweist.

3. Stumpfe Kanüleneinrichtung nach Anspruch 1 oder 2,
wobei das Durchdringungselement einen verjüngten Endbereich angrenzend an sein stumpfes Ende hat.

4. Stumpfe Kanüleneinrichtung nach einem der vorhergehenden Ansprüche, das einstückig aus Kunststoffmaterial hergestellt ist.

5. Stumpfe Kanüleneinrichtung nach einem der vorhergehenden Ansprüche in Kombination mit einer Injektionsstelle (34), die ein erstes Ende, das mit dem Septum (52) versehen ist, und ein zweites Ende (44) hat, mit dem die Arretierelemente in Eingriff bringbar sind.

## Revendications

1. Dispositif de canule utilisable avec un site d 'injection (34) à septum (52), le dispositif comprenant un corps tubulaire (92a), un élément de perçage cylindrique allongé creux (98a) et deux éléments de verrouillage (100a,100b), chaque élément de verrouillage (100a,100b) étant un bras flexible semblable à un ressort actionnable manuellement comportant des moyens de prise manuelle (103) à une extrémité et des moyens (100c) prévus à l'extrémité opposée pour venir en prise avec le site d'injection lorsque l'élément de perçage est inséré dans le septum, afin d'empêcher l'élément de perçage de sortir accidentellement du septum, caractérisé en ce qu'il comprend une traverse (101) d'un côté de laquelle s'étend la partie de corps (92a) et de l'autre côté de laquelle s'étend l'élément de perçage (98a),de sorte que la traverse (101) s'étend radialement entre la partie de corps et l'élément de perçage, chaque élément de verrouillage (100a, 100b) étant fixé, entre ses extrémités, à une extrémité respective de la traverse (101), et l'élément de perçage (98a) présente une extrémité émoussée pour insertion dans un septum préincisé.

2. Dispositif de canule émoussée suivant la revendication 1, dans lequel les moyens (100c) d'accouplement avec le site d'injection comprennent une région d'extrémité courbe en forme de crochet de chaque élément de verrouillage (100a).

3. Dispositif de canule émoussée suivant la revendication 1 ou 2, dans lequel l'élément de perçage a une partie d'extrémité conique adjacente à son extrémité émoussée.

4. Dispositif de canule émoussée suivant une quelconque des revendications précédentes, fabriqué en matière plastique en une seule pièce.

5. Dispositif de canule émoussée suivant une quelconque des revendications précédentes, en combinaison avec un site d'injection (34) ayant une première extrémité munie du septum (52) et une deuxième extrémité (44) avec laquelle les éléments de verrouillage peuvent venir en prise.
